# EUROPEAN PATENT SPECIFICATION

(11) **EP 3 277 881 B1**
(45) Date of publication and mention of the grant of the patent: **24.02.2021**
(21) Application number: 16717739.3
(22) Date of filing: 30.03.2016
(51) Int. Cl.: D06N 3/00, A61F 13/537

(54) **HETEROGENEOUS MASS CONTAINING FOAM**
HETEROGENE MASSE MIT SCHAUMSTOFF
MASSE HÉTÉROGÈNE CONTENANT DE LA MOUSSE

(30) Priority: 31.03.2015 US 201562140801 P
(43) Date of publication of application: 07.02.2018
(73) Proprietor: The Procter & Gamble Company, Cincinnati, OH 45202 (US)
(72) Inventor: HUBBARD, Wade, Monroe, Jr., Cincinnati, Ohio 45202 (US); BEWICK-SONNTAG, Christopher, Philip, Cincinnati, Ohio 45202 (US)
(74) Representative: P&G Patent Germany
(86) International application number: PCT/US2016/024862
(87) International publication number: WO 2016/160900

(56) References cited:
- WO-A1-01/15644
- WO-A1-97/32612
- US-A1- 2012 108 692

## Description

### FIELD OF THE INVENTION

The present invention relates to absorbent structures useful in absorbent articles such as diapers, incontinent briefs, training pants, diaper holders and liners, sanitary hygiene garments, and the like. Specifically, the present invention relates to an absorbent structure having three or more different pore-size ranges wherein two of the pore-size ranges are located within one or more pieces of foam.

### BACKGROUND OF THE INVENTION

WO01/15644A1 discloses an absorbent structure made of compressed foam material which expands upon wetting at which the foam material comprises at least two integrated layers having different mean pore sizes. The layers are not made of a polymerized foam but are made of compressed regenerated cellulose (viscose). While polymeric foams as an option are briefly mentioned WO01/15644A1 discloses stacked layers of compressed foam without any integration with an enrobeable elements as now claimed by in-situ polymerization as now claimed.

WO97/32612 discloses heterogenous foam material having repeating stripes of regions having smaller and larger celled material. WO97/32612 does not disclose polymerizing the open-cell foam directly around enrobeable elements. Rather the foams of WO97/32612 are made of different foam material that are pre-made and stacked according to a desired pattern, and optionally wrapped in a nonwoven for example.

Open celled foams are used for their absorbent properties. Open celled foams include latex polymer foams, polyurethane foams, and foams created by polymerizing an emulsion. One type of an open celled foam is created from an emulsion that is a dispersion of one liquid in another liquid and generally is in the form of a water-in-oil mixture having an aqueous or water phase dispersed within a substantially immiscible continuous oil phase. Water-in-oil (or oil in water) emulsions having a high ratio of dispersed phase to continuous phase are known in the art as High Internal Phase Emulsions, also referred to as "HIPE" or HIPEs. Different foams may be chosen due to specific properties.

Traditionally, open celled foams are polymerized in a continuous sheet or in a tubular reaction. Either process represents that one must use polymerized open celled foam in a continuous form or break up the polymerized open celled foam to make open celled foam pieces.

During processing, one layer of foam may be laid down upon a different layer of foam. This may be done to create a core that has two different pore-size ranges. One pore size may be optimized for acquisition while the other pore size may be optimized for storage.

Ultimately, in regards to an absorbent core, the current process represents using a core made solely of foam or a core that uses pieces of foam placed into or onto another material. If a core with two different pore sizes is desired, this represents having two different processes, each targeting the desired pore size.

Therefore there exists a need to create a foam that has different pore-size ranges within the same foam without using either two different compositions or two different systems that are combined to create the dual pore size structure. Further, there exists a need to go beyond the two pore system and create an absorbent structure that has more than two pore-size ranges within the structure.

### SUMMARY OF THE INVENTION

A heterogeneous mass is disclosed. The heterogeneous mass has a longitudinal axis, a lateral axis, a vertical axis, one or more enrobeable elements and one or more discrete open-cell foam pieces. The heterogeneous mass comprises of three or more pore-size ranges, wherein at least two of the pore-size ranges are in one or more open-cell foam pieces. The open-cell foam is a thermoset polymeric foam made from polymerization of a High Internal Phase Emulsion (HIPE) formed by combining an aqueous phase and an oil phase in a ratio between about 8:1 and 140:1, and the one or more discrete open-cell foam pieces are polymerized around the enrobeable elements.

The open-cell foam may preferably exhibit two distinct pore-size ranges, wherein a first pore-size range is between 100 nanometers and 100 microns and a second pore-size range is between 200 and 5000 microns.

### BRIEF DESCRIPTION OF THE DRAWINGS

While the specification concludes with claims particularly pointing out and distinctly claiming the subject matter of the present invention, it is believed that the invention can be more readily understood from the following description taken in connection with the accompanying drawings, in which:
FIG. 1 is a top view of an absorbent article.
FIG. 2 is a cross section view of the absorbent article of FIG. 1 taken along line 2-2.
FIG. 3 is a cross section view of the absorbent article of FIG. 1 taken along line 3-3.
FIG. 4 is a top view of an absorbent article.
FIG. 5 is a cross section view of the absorbent article of FIG. 4 taken along line 5-5.
FIG. 6 is a cross section view of the absorbent article of FIG. 4 taken along line 6-6.
FIG. 7 is a cross section view of the absorbent article of FIG. 4 taken along line 7-7.
FIG. 8 is a magnified view of a portion of FIG. 5.
FIG. 9 is a top view of an absorbent article.
FIG. 10 is a cross section view of the absorbent article of FIG. 9 taken along line 10-10.
FIG. 11 is a cross section view of the absorbent article of FIG. 9 taken along line 11-11.
FIG. 12 is an SEM of a representative HIPE foam piece.
FIG. 13 is a magnified view of the SEM of FIG. 12.

### DETAILED DESCRIPTION OF THE INVENTION

As used herein, the term "bicomponent fibers" refers to fibers which have been formed from at least two different polymers extruded from separate extruders but spun together to form one fiber. Bicomponent fibers are also sometimes referred to as conjugate fibers or multicomponent fibers. The polymers are arranged in substantially constantly positioned distinct zones across the cross-section of the bicomponent fibers and extend continuously along the length of the bicomponent fibers. The configuration of such a bicomponent fiber may be, for example, a sheath/core arrangement wherein one polymer is surrounded by another, or may be a side-by-side arrangement, a pie arrangement, or an "islands-in-the-sea" arrangement.

As used herein, the term "biconstituent fibers" refers to fibers which have been formed from at least two polymers extruded from the same extruder as a blend. Biconstituent fibers do not have the various polymer components arranged in relatively constantly positioned distinct zones across the cross-sectional area of the fiber and the various polymers are usually not continuous along the entire length of the fiber, instead usually forming fibrils which start and end at random. Biconstituent fibers are sometimes also referred to as multiconstituent fibers.

The term "disposable" is used herein to describe articles, which are not intended to be laundered or otherwise restored or reused as an article (i.e. they are intended to be discarded after a single use and possibly to be recycled, composted or otherwise disposed of in an environmentally compatible manner). The absorbent article comprising an absorbent structure according to the present invention can be for example a sanitary napkin or a panty liner. The absorbent structure of the present invention will be herein described in the context of a typical absorbent article, such as, for example, a sanitary napkin. Typically, such articles can comprise a liquid pervious topsheet, a backsheet and an absorbent core intermediate the topsheet and the backsheet.

As used herein, an "enrobeable element" refers to an element that may be enrobed by the foam. The enrobeable element may be, for example, a fiber, a group of fibers, a tuft, or a section of a film between two apertures. It is understood that other elements are contemplated by the present invention.

A "fiber" as used herein, refers to any material that can be part of a fibrous structure. Fibers can be natural or synthetic. Fibers can be absorbent or non-absorbent.

A "fibrous structure" as used herein, refers to materials which can be broken into one or more fibers. A fibrous structure can be absorbent or adsorbent. A fibrous structure can exhibit capillary action as well as porosity and permeability.

As used herein, the term "meltblowing" refers to a process in which fibers are formed by extruding a molten thermoplastic material through a plurality of fine, usually circular, die capillaries as molten threads or filaments into converging high velocity, usually heated, gas (for example air) streams which attenuate the filaments of molten thermoplastic material to reduce their diameter. Thereafter, the meltblown fibers are carried by the high velocity gas stream and are deposited on a collecting surface, often while still tacky, to form a web of randomly dispersed meltblown fibers.

As used herein, the term "monocomponent" fiber refers to a fiber formed from one or more extruders using only one polymer. This is not meant to exclude fibers formed from one polymer to which small amounts of additives have been added for coloration, antistatic properties, lubrication, hydrophilicity, etc. These additives, for example titanium dioxide for coloration, are generally present in an amount less than about 5 weight percent and more typically about 2 weight percent.

As used herein, the term "non-round fibers" describes fibers having a non-round cross-section, and includes "shaped fibers" and "capillary channel fibers." Such fibers can be solid or hollow, and they can be tri-lobal, delta-shaped, or can be fibers having capillary channels on their outer surfaces. The capillary channels can be of various cross-sectional shapes such as "U-shaped", "H-shaped", "C-shaped" and "V-shaped". One practical capillary channel fiber is T-401, designated as 4DG fiber available from Fiber Innovation Technologies, Johnson City, TN. T-401 fiber is a polyethylene terephthalate (PET polyester).

As used herein, the term "nonwoven web" refers to a web having a structure of individual fibers or threads which are interlaid, but not in a repeating pattern as in a woven or knitted fabric, which do not typically have randomly oriented fibers. Nonwoven webs or fabrics have been formed from many processes, such as, for example, meltblowing processes, spunbonding processes, spunlacing processes, hydroentangling, airlaying, and bonded carded web processes, including carded thermal bonding. The basis weight of nonwoven fabrics is usually expressed in grams per square meter (gsm). The basis weight of the laminate web is the combined basis weight of the constituent layers and any other added components. Fiber diameters are usually expressed in microns; fiber size can also be expressed in denier, which is a unit of weight per length of fiber. The basis weight of laminate webs suitable for use in an article of the present invention can range from 10 gsm to 100 gsm, depending on the ultimate use of the web.

As used herein, the term "polymer" generally includes, but is not limited to, homopolymers, copolymers, such as for example, block, graft, random and alternating copolymers, terpolymers, etc., and blends and modifications thereof. In addition, unless otherwise specifically limited, the term "polymer" includes all possible geometric configurations of the material. The configurations include, but are not limited to, isotactic, atactic, syndiotactic, and random symmetries.

As used herein, "spunbond fibers" refers to small diameter fibers which are formed by extruding molten thermoplastic material as filaments from a plurality of fine, usually circular capillaries of a spinneret with the diameter of the extruded filaments then being rapidly reduced. Spunbond fibers are generally not tacky when they are deposited on a collecting surface. Spunbond fibers are generally continuous and have average diameters (from a sample size of at least 10 fibers) larger than 7 microns, and more particularly, between about 10 and 40 microns.

As used herein, a "tuft" or chad relates to discrete integral extensions of the fibers of a nonwoven web. Each tuft can comprise a plurality of looped, aligned fibers extending outwardly from the surface of the web. In another embodiment each tuft can comprise a plurality of non-looped fibers that extend outwardly from the surface of the web. In another embodiment, each tuft can comprise a plurality of fibers which are integral extensions of the fibers of two or more integrated nonwoven webs.

### GENERAL SUMMARY

The present invention relates to an absorbent structure that is a heterogeneous mass as defined in appended claim 1, comprising one or more enrobeable elements and one or more discrete open-cell foam pieces. The heterogeneous mass has at least three distinct pore-size ranges. Two of the three pore-size ranges are located within the foam pieces. The foam pieces have a first pore-size range at one surface and a second pore-size range at a second surface. At least one of the two pore-size ranges is greater than the cross sectional area of the smallest enrobeable element. The heterogeneous mass has a depth, a width, and a height. The absorbent structure may be used as any part of an absorbent article including, for example, a part of an absorbent core, as an absorbent core, and/or as a topsheet for absorbent articles such as sanitary napkins, panty liners, tampons, interlabial devices, wound dressings, diapers, adult incontinence articles, and the like, which are intended for the absorption of body fluids, such as menses or blood or vaginal discharges or urine. The absorbent structure may be used in any product utilized to absorb and retain a fluid including surface wipes. The absorbent structure may be used as a paper towel. Exemplary absorbent articles in the context of the present invention are disposable absorbent articles.

The absorbent structure is a heterogeneous mass comprising enrobeable elements and one or more discrete portions of foam pieces. The one or more discrete portions of foam pieces enrobe the elements. The discrete portions of foam pieces are open celled foam. The foam is a High Internal Phase Emulsion (HIPE) foam.

In an embodiment, the absorbent structure is an absorbent core for an absorbent article wherein the absorbent core comprises a heterogeneous mass comprising fibers and one or more discrete portions of foam that enrobe one or more of the fibers.

In the following description of the invention, the surface of the article, or of each component thereof, which in use faces in the direction of the wearer is called wearer-facing surface. Conversely, the surface facing in use in the direction of the garment is called garment-facing surface. The absorbent article of the present invention, as well as any element thereof, such as, for example the absorbent core, has therefore a wearer-facing surface and a garment-facing surface.

The present invention relates to an absorbent structure that contains one or more discrete open-cell foam pieces foams that are integrated into a heterogeneous mass comprising one or more enrobeable elements integrated into the one or more open-cell foams such that the two may be intertwined.

The open-cell foam pieces may comprise between 1% of the heterogeneous mass by volume to 99% of the heterogeneous mass by volume, such as, for example, 5% by volume, 10% by volume, 15% by volume, 20% by volume, 25% by volume, 30% by volume, 35% by volume, 40% by volume, 45% by volume, 50% by volume, 55% by volume, 60% by volume, 65% by volume, 70% by volume, 75% by volume, 80% by volume, 85% by volume, 90% by volume, or 95% by volume.

The heterogeneous mass may have void space found between the enrobeable elements, between the enrobeable elements and the enrobed elements, and between enrobed elements. The void space may contain gas. The void space may represent between 1% and 95% of the total volume for a fixed amount of volume of the heterogeneous mass, such as, for example, 5%, 10%, 15%, 20%, 25%, 30%, 35%, 40%, 45%, 50%, 55%, 60%, 65%, 70%, 75%, 80%, 85%, 90% of the total volume for a fixed amount of volume of the heterogeneous mass.

The combination of open-cell foam pieces and void space within the heterogeneous mass may exhibit an absorbency of between 10 g/g to 200 g/g of the heterogeneous mass, such as for example, 40 g/g, 60 g/g, 80 g/g, 100 g/g, 120 g/g, 140 g/g 160 g/g 180 g/g or 190 g/g of the heterogeneous mass. Absorbency may be quantified according to the EDANA Nonwoven Absorption method 10.4-02.

In an embodiment, the heterogeneous mass exhibits three or more pore-size ranges wherein at least a first pore-size range and a second pore-size range are located within one or more of the open-cell foam pieces. The enrobeable elements within the heterogeneous mass may have a pore size. The pore size of the enrobeable elements may be between 200 and 5,000 microns, such as, for example, between 250 and 4,000 microns, between 300 and 3,500 microns, between 350 and 3,000 microns, between 500 and 2,500 microns, between 1,000 and 2,000 microns. In an embodiment, the pore-size average of the enrobeable elements within the heterogeneous mass may be between 200 and 5,000 microns, such as, for example, between 250 and 4,000 microns, between 300 and 3,500 microns, between 350 and 3,000 microns, between 500 and 2,500 microns, between 1,000 and 2,000 microns.

In an embodiment, the open-cell foam pieces have two distinct pore-size ranges such as, for example, a first pore-size range of 5 microns to 100 microns and a second pore-size range of about 200 microns to about 5000 microns. In an embodiment, the first pore-size range may be 5 microns to 100 microns, 10 microns to 90 microns, 25 microns to 75 microns. In an embodiment, the second pore-size range may be 250 microns to 900 microns, 300 microns to 800 microns, 400 microns to 700 microns, 500 microns to 600 microns. The second pore size of the open-cell foam piece may be between 200 and 5,000 microns, such as, for example, between 250 and 4,000 microns, between 300 and 3,500 microns, between 350 and 3,000 microns, between 500 and 2,500 microns, between 1,000 and 2,000 microns. In an embodiment, the second pore-size average of the open-cell foam within the heterogeneous mass may be between 200 and 5,000 microns, such as, for example, between 250 and 4,000 microns, between 300 and 3,500 microns, between 350 and 3,000 microns, between 500 and 2,500 microns, between 1,000 and 2,000 microns.

In an embodiment, a distinct piece of foam may exhibit a first pore-size range at a first surface and a second pore-size range at a second surface that is opposite the first surface along either the longitudinal, transverse, or vertical axis of the distinct piece of foam. In an embodiment, a piece of foam may exhibit a first pore-size range in a first section of the foam piece and a second pore-size range in second section of the foam piece. The first section and the second section may be separated by a visual demarcation.

In an embodiment, the open-cell foam piece gradates from the first pore-size range to the second pore-size range such that the pore size transitions within the foam piece from a first pore-size range to a second pore-size range. The open-cell foam piece may gradate from the first open pore-size range to the second open pore-size range along the lateral, vertical, or longitudinal axis. In an embodiment, the open-cell foam piece may gradate from a first open pore-size range to a second open pore-size range and back to the first open pore-size range along any of the lateral, longitudinal, or vertical axis. In an embodiment, the pore-size average at a second end of an axis is a multiple of the pore-size average at a first end of an axis, such as, for example, between 1.1 and 100 times, between 10 and 90 times, between 20 and 80 times.

In an embodiment, the foam piece may have a third pore-size range located between the first pore size and the second pore size. The third pore-size range may be located in the section that separates the first foam piece section and the second foam piece section.

In an embodiment, the second pore-size range may be of sufficient size to enrobe an enrobeable element within the pore.

The open-cell foam pieces are discrete foam pieces intertwined within and throughout a heterogeneous mass such that the open-cell foam enrobes one or more of the enrobeable elements such as, for example, fibers within the mass. The open-cell foam may be polymerized around the enrobeable elements.

In an embodiment, a discrete open-cell foam piece may enrobe more than one enrobeable element. The enrobeable elements may be enrobed together as a bunch. Alternatively, more than one enrobeable element may be enrobed by the discrete open-cell foam piece without contacting another enrobeable element.

In an embodiment, the open-cell foam pieces may enrobe an enrobeable element such that the enrobeable element is enrobed along the enrobeable elements axis for between 5% and 95% of the length along the enrobeable element's axis. For example, a single fiber may be enrobed along the length of the fiber for a distance greater than 50% of the entire length of the fiber. In an embodiment, an enrobeable element may have between 5% and 100% of its surface area enrobed by one or more open-cell foam pieces.

In an embodiment, two or more open-cell foam pieces may enrobe the same enrobeable element such that the enrobeable element is enrobed along the enrobeable elements axis for between 5% and 100% of the length along the enrobeable element's axis.

The open-cell foam pieces enrobe the enrobeable elements such that a layer surrounds the enrobeable element at a given cross section. The layer surrounding the enrobeable element at a given cross section may be between 0.01 mm to 100 mm such as, for example, 0.1 mm, 0.2 mm, 0.3 mm, 0.4 mm, 0.5 mm, 0.6 mm, 0.7 mm, 0.8 mm, 0.9 mm, 1.0 mm, 1.2 mm, 1.4 mm, 1.6 mm, 1.8 mm, 2.0 mm, 2.2 mm, 2.4 mm, 2.6 mm, 2.8 mm, or 3 mm. The layer may not be equivalent in dimension at all points along the cross section of the enrobeable element. For example, in an embodiment, an enrobeable element may be enrobed by 0.5 mm at one point along the cross section and by 1.0 mm at a different point along the same cross section.

The open-cell foam pieces are considered discrete in that they are not continuous throughout the entire heterogeneous mass. Not continuous throughout the entire heterogeneous mass represents that at any given point in the heterogeneous mass, the open cell absorbent foam is not continuous in at least one of the cross sections of a longitudinal, a vertical, and a lateral plane of the heterogeneous mass. In a non-limiting embodiment, the absorbent foam is not continuous in the lateral and the vertical planes of the cross section for a given point in the heterogeneous mass. In a non-limiting embodiment, the absorbent foam is not continuous in the longitudinal and the vertical planes of the cross section for a given point in the heterogeneous mass. In a non-limiting embodiment, the absorbent foam is not continuous in the longitudinal and the lateral planes of the cross section for a given point in the heterogeneous mass.

In an embodiment wherein the open-cell foam is not continuous in at least one of the cross sections of the longitudinal, the vertical, and the lateral plane of the heterogeneous mass, one or both of either the enrobeable elements or the open-cell foam pieces may be bi-continuous throughout the heterogeneous mass.

The open-cell foam pieces may be located at any point in the heterogeneous mass. In a non-limiting embodiment, a foam piece may be surrounded by the elements that make up the enrobeable elements. In a non-limiting embodiment a foam piece may be located on the outer perimeter of the heterogeneous mass such that only a portion of the foam piece is entangled with the elements of the heterogeneous mass.

In a non-limiting embodiment, the open-cell foam pieces may expand upon being contacted by a fluid to form a channel of discrete open-cell foam pieces. The open-cell foam pieces may or may not be in contact prior to being expanded by a fluid.

An open celled foam is integrated onto the enrobeable elements prior to being polymerized. In a non-limiting embodiment the open-cell foam pieces may be partially polymerized prior to being impregnated into or onto the enrobeable elements such that they become intertwined. After being impregnated into or onto the enrobeable elements, the open celled foam in either a liquid or solid state are polymerized to form one or more open-cell foam pieces. The open celled foam may be polymerized using any known method including, for example, heat, UV, and infrared. Following the polymerization of a water in oil emulsion, the resulting open-cell foam is saturated with aqueous phase that needs to be removed to obtain a substantially dry open-cell foam. Removal of the saturated aqueous phase or dewatering may occur using nip rollers, and vacuum. Utilizing a nip roller may also reduce the thickness of the heterogeneous mass such that the heterogeneous mass will remain thin until the open-cell foam pieces entwined in the heterogeneous mass are exposed to fluid.

The open-cell foam pieces may enrobe the enrobeable elements in a manner that creates a spacing or vacuole between the enrobing foam and the enrobeable element. The vacuole contains the enrobeable element and may surround the entire element, a cross section of the element, or a portion of the element. In an embodiment, the open-cell foam pieces may be in direct contact with the element at one location and spaced by a vacuole in another. The vacuole may allow the enrobeable element to move within the vacuole. The size of the vacuole may be driven by the type of enrobeable element. In an embodiment, the vacuole diameter is greater than the fiber diameter which is greater than the foam pore size. The vacuole diameter may be, for example, between 1.0001 and 30,000 times the diameter of the fiber diameter, such as, between 1.2 and 20,000 times the diameter of the fiber, 10 and 10,000 times the diameter of the fiber, 100 and 1,000, such as, for example, 20times the diameter of the fiber, 150 times the diameter of the fiber, 1,500 times the diameter of the fiber, 3,000 times the diameter of the fiber, 4,500 times the diameter of the fiber, 6,000 times the diameter of the fiber, 7,500 times the diameter of the fiber, 9,000 times the diameter of the fiber, 12,000 times the diameter of the fiber, 15,000 times the diameter of the fiber, 18,000 times the diameter of the fiber, 21,000 times the diameter of the fiber, 24,000 times the diameter of the fiber, 27,000, or 29,000 times the diameter of the fiber.

In an embodiment, one or more vacuoles may be irregularly shaped. In such embodiments, the cross-sectional surface area of the vacuoles may be between 1.0002 and 900,000,000 times the surface area created by a cross section of the fiber. When more than one fiber is located in the same vacuole, the cross-sectional surface area of the vacuoles may be between 1.0002 and 900,000,000 times the surface area created by the sum of the cross section of the fibers, such as, for example, between 10 to 100,000,000 times the surface area created by the sum of the cross section of the fibers, between 1,000 to 1,000,000 times the surface area created by the sum of the cross section of the fibers, or between 10,000 to 100,000 times the surface area created by the sum of the cross section of the fibers.

In an embodiment, the cross-sectional surface area of the vacuoles may be between 1.26 and 9,000,000 times the cross-sectional surface area of the pores in the open-cell foam such as, for example between 100 and 5,000,000 times the cross-sectional surface area of the pores in the open-cell foam, between 1,000 and 1,000,000 times the cross-sectional surface area of the pores in the open-cell foam, between 100,000 and 500,000 times the cross-sectional surface area of the pores in the open-cell foam. The cross sectional area of the pores may be between 0.001% and 99.99% of the cross sectional area of the vacuoles. The cross-sectional surface area of the vacuoles, pores (also referred to as cells) of the open-cell foams, and fiber diameters are measured via quantitative image analysis of cross-sectional micrographs of the heterogeneous mass.

Dependent upon the desired foam density, polymer composition, specific surface area, or pore size (also referred to as cell size), the open celled foam may be made with different chemical composition, physical properties, or both. For instance, dependent upon the chemical composition, an open celled foam may have a density of 0.0010 g/cc to about 0.25 g/cc, or from 0.002 g/cc to about 0.2 g/cc, or from about 0.005 g/cc to about 0.15 g/cc, or from about 0.01 g/cc to about 0.1 g/cc, or from about 0.02 g/cc to about 0.08 g/cc, or about 0.04 g/cc.

The foams produced from the present invention are relatively open-celled. This refers to the individual cells or pores of the foam being in substantially unobstructed communication with adjoining cells. The cells in such substantially open-celled foam structures have intercellular openings or windows that are large enough to permit ready fluid transfer from one cell to another within the foam structure. For purpose of the present invention, a foam is considered "open-celled" if at least about 80% of the cells in the foam that are at least 1µm in average diameter size are in fluid communication with at least one adjoining cell.

In addition to being open-celled, in certain embodiments foams are sufficiently hydrophilic to permit the foam to absorb aqueous fluids, for example the internal surfaces of a foam may be rendered hydrophilic by residual emulsifiers, hydrophilizing surfactants, or salts left in the foam following polymerization, by selected post-polymerization foam treatment procedures (as described hereafter), or combinations of both.

In certain embodiments, for example when used in certain absorbent articles, an open-cell foam may be flexible and exhibit an appropriate glass transition temperature (Tg). The Tg represents the midpoint of the transition between the glassy and rubbery states of the polymer.

In certain embodiments, the Tg of this region will be less than about 200° C for foams used at about ambient temperature conditions, in certain other embodiments less than about 90° C. The Tg may be less than 50° C.

The open-cell foam pieces may be distributed in any suitable manner throughout the heterogeneous mass. In an embodiment, the open-cell foam pieces may be profiled along the vertical axis such that smaller pieces are located above larger pieces. Alternatively, the pieces may be profiled such that smaller pieces are below larger pieces. In another embodiment, the open cell pieces may be profiled along a vertical axis such that they alternate in size along the axis.

In an embodiment, the open-cell foam pieces may be profiled along the longitudinal axis such that smaller pieces are located in front of larger pieces. Alternatively, the pieces may be profiled such that smaller pieces are behind larger pieces. In another embodiment, the open cell pieces may be profiled along a longitudinal axis such that they alternate in size along the axis.

In an embodiment, the open-cell foam pieces may be profiled along the lateral axis such the size of the pieces goes from small to large or from large to small along the lateral axis. Alternatively, the open cell pieces may be profiled along a lateral axis such that they alternate in size along the axis.

In an embodiment the open-cell foam pieces may be profiled along any one of the longitudinal, lateral, or vertical axis based on one or more characteristics of the open-cell foam pieces. Characteristics by which the open-cell foam pieces may be profiled within the heterogeneous mass may include, for example, absorbency, density, cell size, and combinations thereof.

In an embodiment, the open-cell foam pieces may be profiled along any one of the longitudinal, lateral, or vertical axis based on the composition of the open-cell foam. The open-cell foam pieces may have one composition exhibiting desirable characteristics in the front of the heterogeneous mass and a different composition in the back of the heterogeneous mass designed to exhibit different characteristics. The profiling of the open-cell foam pieces may be either symmetric or asymmetric about any of the prior mentioned axes or orientations.

The open-cell foam pieces may be distributed along the longitudinal and lateral axis of the heterogeneous mass in any suitable form. In an embodiment, the open-cell foam pieces may be distributed in a manner that forms a design or shape when viewed from a top planar view. The open-cell foam pieces may be distributed in a manner that forms stripes, ellipticals, squares, or any other known shape or pattern.

The distribution may be optimized dependent on the intended use of the heterogeneous mass. For example, a different distribution may be chosen for the absorption of aqueous fluids such as urine when used in a diaper or water when used in a paper towel versus for the absorption of a proteinaceous fluid such as menses. Further, the distribution may be optimized for uses such as dosing an active or to use the foam as a reinforcing element.

The pieces may be located at specific locations within the mass based on their properties to optimize the performance of the heterogeneous mass.

In an embodiment, the foam pieces and enrobeable elements may be selected to complement each other. For example, a foam that exhibits high permeability with low capillarity may enrobe an element that exhibits high capillarity to wick the fluid through the heterogeneous mass. It is understood that other combinations may be possible wherein the foam pieces complement each other or wherein the foam pieces and enrobeable elements both exhibit similar properties.

In an embodiment, profiling may occur using more than one heterogeneous mass with each heterogeneous mass having one or more types of foam pieces. The plurality of heterogeneous masses may be layered so that the foam is profiled along any one of the longitudinal, lateral, or vertical axis based on one or more characteristics of the open-cell foam pieces for an overall product that contains the plurality of heterogeneous masses. Further, each heterogeneous mass may have a different enrobeable element to which the foam is attached. For example, a first heterogeneous mass may have foam particles enrobing a nonwoven while a second heterogeneous mass adjacent the first heterogeneous mass may have foam particles enrobing a film or one surface of a film.

The open celled foam is a thermoset polymeric foam made from the polymerization of a High Internal Phase Emulsion (HIPE), also referred to as a polyHIPE. To form a HIPE, an aqueous phase and an oil phase are combined in a ratio between about 8:1 and 140:1. In certain embodiments, the aqueous phase to oil phase ratio is between about 10:1 and about 75:1, and in certain other embodiments the aqueous phase to oil phase ratio is between about 13:1 and about 65:1. This is termed the "water-to-oil" or W:O ratio and can be used to determine the density of the resulting polyHIPE foam. As discussed, the oil phase may contain one or more of monomers, comonomers, photoinitiators, crosslinkers, and emulsifiers, as well as optional components. The water phase will contain water and in certain embodiments one or more components such as electrolyte, initiator, or optional components.

The open-cell foam can be formed from the combined aqueous and oil phases by subjecting these combined phases to shear agitation in a mixing chamber or mixing zone. The combined aqueous and oil phases are subjected to shear agitation to produce a stable HIPE having aqueous droplets of the desired size. An initiator may be present in the aqueous phase, or an initiator may be introduced during the foam making process, and in certain embodiments, after the HIPE has been formed. The emulsion making process produces a HIPE where the aqueous phase droplets are dispersed to such an extent that the resulting HIPE foam will have the desired structural characteristics. Emulsification of the aqueous and oil phase combination in the mixing zone may involve the use of a mixing or agitation device such as an impeller, by passing the combined aqueous and oil phases through a series of static mixers at a rate necessary to impart the requisite shear, or combinations of both. Once formed, the HIPE can then be withdrawn or pumped from the mixing zone. One method for forming HIPEs using a continuous process is described in U.S. Pat. No. 5,149,720 (DesMarais et al), issued Sep. 22, 1992; U.S. Pat. No. 5,827,909 (DesMarais) issued Oct. 27, 1998; and U.S. Pat. No. 6,369,121 (Catalfamo et al.) issued Apr. 9, 2002.

The emulsion can be withdrawn or pumped from the mixing zone and impregnated into or onto a mass prior to being fully polymerized. Once fully polymerized, the foam pieces and the elements are intertwined such that discrete foam pieces are bisected by the elements comprising the mass and such that parts of discrete foam pieces enrobe portions of one or more of the elements comprising the heterogeneous mass.

Following polymerization, the resulting foam pieces are saturated with aqueous phase that needs to be removed to obtain substantially dry foam pieces. In certain embodiments, foam pieces can be squeezed free of most of the aqueous phase by using compression, for example by running the heterogeneous mass comprising the foam pieces through one or more pairs of nip rollers. The nip rollers can be positioned such that they squeeze the aqueous phase out of the foam pieces. The nip rollers can be porous and have a vacuum applied from the inside such that they assist in drawing aqueous phase out of the foam pieces. In certain embodiments, nip rollers can be positioned in pairs, such that a first nip roller is located above a liquid permeable belt, such as a belt having pores or composed of a mesh-like material and a second opposing nip roller facing the first nip roller and located below the liquid permeable belt. One of the pair, for example the first nip roller can be pressurized while the other, for example the second nip roller, can be evacuated, so as to both blow and draw the aqueous phase out the of the foam. The nip rollers may also be heated to assist in removing the aqueous phase. In certain embodiments, nip rollers are only applied to non-rigid foams, that is, foams whose walls would not be destroyed by compressing the foam pieces.

In certain embodiments, in place of or in combination with nip rollers, the aqueous phase may be removed by sending the foam pieces through a drying zone where it is heated, exposed to a vacuum, or a combination of heat and vacuum exposure. Heat can be applied, for example, by running the foam though a forced air oven, IR oven, microwave oven or radiowave oven. The extent to which a foam is dried depends on the application. In certain embodiments, greater than 50% of the aqueous phase is removed. In certain other embodiments greater than 90%, and in still other embodiments greater than 95% of the aqueous phase is removed during the drying process.

In an embodiment, open-cell foam is produced from the polymerization of the monomers having a continuous oil phase of a High Internal Phase Emulsion (HIPE). The HIPE may have two phases. One phase is a continuous oil phase having monomers that are polymerized to form a HIPE foam and an emulsifier to help stabilize the HIPE. The oil phase may also include one or more photoinitiators. The monomer component may be present in an amount of from about 80% to about 99%, and in certain embodiments from about 85% to about 95% by weight of the oil phase. The emulsifier component, which is soluble in the oil phase and suitable for forming a stable water-in-oil emulsion may be present in the oil phase in an amount of from about 1% to about 20% by weight of the oil phase. The emulsion may be formed at an emulsification temperature of from about 10° C to about 130° C and in certain embodiments from about 50° C to about 100° C.

In general, the monomers will include from about 20% to about 97% by weight of the oil phase at least one substantially water-insoluble monofunctional alkyl acrylate or alkyl methacrylate. For example, monomers of this type may include C₄-C₁₈ alkyl acrylates and C₂-C₁₈ methacrylates, such as ethylhexyl acrylate, butyl acrylate, hexyl acrylate, octyl acrylate, nonyl acrylate, decyl acrylate, isodecyl acrylate, tetradecyl acrylate, benzyl acrylate, nonyl phenyl acrylate, hexyl methacrylate, 2-ethylhexyl methacrylate, octyl methacrylate, nonyl methacrylate, decyl methacrylate, isodecyl methacrylate, dodecyl methacrylate, tetradecyl methacrylate, and octadecyl methacrylate.

The oil phase may also have from about 2% to about 40%, and in certain embodiments from about 10% to about 30%, by weight of the oil phase, a substantially water-insoluble, polyfunctional crosslinking alkyl acrylate or methacrylate. This crosslinking comonomer, or crosslinker, is added to confer strength and resilience to the resulting HIPE foam. Examples of crosslinking monomers of this type may have monomers containing two or more activated acrylate, methacrylate groups, or combinations thereof. Nonlimiting examples of this group include 1,6-hexanedioldiacrylate, 1,4-butanedioldimethacrylate, trimethylolpropane triacrylate, trimethylolpropane trimethacrylate, 1,12-dodecyldimethacrylate, 1,14-tetradecanedioldimethacrylate, ethylene glycol dimethacrylate, neopentyl glycol diacrylate (2,2-dimethylpropanediol diacrylate), hexanediol acrylate methacrylate, glucose pentaacrylate, sorbitan pentaacrylate, and the like. Other examples of crosslinkers contain a mixture of acrylate and methacrylate moieties, such as ethylene glycol acrylate-methacrylate and neopentyl glycol acrylate-methacrylate. The ratio of methacrylate:acrylate group in the mixed crosslinker may be varied from 50:50 to any other ratio as needed.

Any third substantially water-insoluble comonomer may be added to the oil phase in weight percentages of from about 0% to about 15% by weight of the oil phase, in certain embodiments from about 2% to about 8%, to modify properties of the HIPE foams. In certain embodiments, "toughening" monomers may be desired which impart toughness to the resulting HIPE foam. These include monomers such as styrene, vinyl chloride, vinylidene chloride, isoprene, and chloroprene. Without being bound by theory, it is believed that such monomers aid in stabilizing the HIPE during polymerization (also known as "curing") to provide a more homogeneous and better formed HIPE foam which results in better toughness, tensile strength, abrasion resistance, and the like. Monomers may also be added to confer flame retardancy as disclosed in U.S. Pat. No. 6,160,028 (Dyer) issued Dec. 12, 2000. Monomers may be added to confer color, for example vinyl ferrocene, fluorescent properties, radiation resistance, opacity to radiation, for example lead tetraacrylate, to disperse charge, to reflect incident infrared light, to absorb radio waves, to form a wettable surface on the HIPE foam struts, or for any other desired property in a HIPE foam. In some cases, these additional monomers may slow the overall process of conversion of HIPE to HIPE foam, the tradeoff being necessary if the desired property is to be conferred. Thus, such monomers can be used to slow down the polymerization rate of a HIPE. Examples of monomers of this type can have styrene and vinyl chloride.

The oil phase may further contain an emulsifier used for stabilizing the HIPE. Emulsifiers used in a HIPE can include: (a) sorbitan monoesters of branched C₁₆-C₂₄ fatty acids; linear unsaturated C₁₆-C₂₂ fatty acids; and linear saturated C₁₂-C₁₄ fatty acids, such as sorbitan monooleate, sorbitan monomyristate, and sorbitan monoesters, sorbitan monolaurate diglycerol monooleate (DGMO), polyglycerol monoisostearate (PGMIS), and polyglycerol monomyristate (PGMM); (b) polyglycerol monoesters of -branched C₁₆-C₂₄ fatty acids, linear unsaturated C₁₆-C₂₂ fatty acids, or linear saturated C₁₂-C₁₄ fatty acids, such as diglycerol monooleate (for example diglycerol monoesters of C18:1 fatty acids), diglycerol monomyristate, diglycerol monoisostearate, and diglycerol monoesters; (c) diglycerol monoaliphatic ethers of -branched C₁₆-C₂₄ alcohols, linear unsaturated C₁₆-C₂₂ alcohols, and linear saturated C₁₂-C₁₄ alcohols, and mixtures of these emulsifiers. See U.S. Pat. No. 5,287,207 (Dyer et al.), issued Feb. 7, 1995 and U.S. Pat. No. 5,500,451 (Goldman et al.) issued Mar. 19, 1996. Another emulsifier that may be used is polyglycerol succinate (PGS), which is formed from an alkyl succinate, glycerol, and triglycerol.

Such emulsifiers, and combinations thereof, may be added to the oil phase so that they can have between about 1% and about 20%, in certain embodiments from about 2% to about 15%, and in certain other embodiments from about 3% to about 12% by weight of the oil phase. In certain embodiments, coemulsifiers may also be used to provide additional control of cell size, cell size distribution, and emulsion stability, particularly at higher temperatures, for example greater than about 65° C. Examples of coemulsifiers include phosphatidyl cholines and phosphatidyl choline-containing compositions, aliphatic betaines, long chain C₁₂-C₂₂ dialiphatic quaternary ammonium salts, short chain C₁-C₄ dialiphatic quaternary ammonium salts, long chain C₁₂-C₂₂ dialkoyl(alkenoyl)-2-hydroxyethyl, short chain C₁-C₄ dialiphatic quaternary ammonium salts, long chain C₁₂-C₂₂ dialiphatic imidazolinium quaternary ammonium salts, short chain C₁-C₄ dialiphatic imidazolinium quaternary ammonium salts, long chain C₁₂-C₂₂ monoaliphatic benzyl quaternary ammonium salts, long chain C₁₂-C₂₂ dialkoyl(alkenoyl)-2-aminoethyl, short chain C₁-C₄ monoaliphatic benzyl quaternary ammonium salts, short chain C₁-C₄ monohydroxyaliphatic quaternary ammonium salts. In certain embodiments, ditallow dimethyl ammonium methyl sulfate (DTDMAMS) may be used as a coemulsifier.

The oil phase may comprise a photoinitiator at between about 0.05% and about 10%, and in certain embodiments between about 0.2% and about 10% by weight of the oil phase. Lower amounts of photoinitiator allow light to better penetrate the HIPE foam, which can provide for polymerization deeper into the HIPE foam. However, if polymerization is done in an oxygen-containing environment, there should be enough photoinitiator to initiate the polymerization and overcome oxygen inhibition. Photoinitiators can respond rapidly and efficiently to a light source with the production of radicals, cations, and other species that are capable of initiating a polymerization reaction. The photoinitiators used in the present invention may absorb UV light at wavelengths of about 200 nanometers (nm) to about 800 nm, in certain embodiments about 200 nm to about 350 nm. If the photoinitiator is in the oil phase, suitable types of oil-soluble photoinitiators include benzyl ketals, α-hydroxyalkyl phenones, α-amino alkyl phenones, and acylphospine oxides. Examples of photoinitiators include 2,4,6-[trimethylbenzoyldiphosphine] oxide in combination with 2-hydroxy-2-methyl-1-phenylpropan-1-one (50:50 blend of the two is sold by Ciba Speciality Chemicals, Ludwigshafen, Germany as DAROCUR® 4265); benzyl dimethyl ketal (sold by Ciba Geigy as IRGACURE 651); α-,α-dimethoxy-a-hydroxy acetophenone (sold by Ciba Speciality Chemicals as DAROCUR® 1173); 2-methyl-1-[4-(methyl thio) phenyl]-2-morpholino-propan-1-one (sold by Ciba Speciality Chemicals as IRGACURE® 907); 1-hydroxycyclohexyl-phenyl ketone (sold by Ciba Speciality Chemicals as IRGACURE® 184); bis(2,4,6-trimethylbenzoyl)-phenylphosphineoxide (sold by Ciba Speciality Chemicals as IRGACURE 819); diethoxyacetophenone, and 4-(2-hydroxyethoxy)phenyl-(2-hydroxy-2-methylpropyl) ketone (sold by Ciba Speciality Chemicals as IRGACURE® 2959); and Oligo [2-hydroxy-2-methyl-1-[4-(1-methylvinyl) phenyl]propanone] (sold by Lamberti spa, Gallarate, Italy as ESACURE® KIP EM.

The dispersed aqueous phase of a HIPE can have water, and may also have one or more components, such as initiator, photoinitiator, or electrolyte, wherein in certain embodiments, the one or more components are at least partially water soluble.

One component of the aqueous phase may be a water-soluble electrolyte. The water phase may contain from about 0.2% to about 40%, in certain embodiments from about 2% to about 20%, by weight of the aqueous phase of a water-soluble electrolyte. The electrolyte minimizes the tendency of monomers, comonomers, and crosslinkers that are primarily oil soluble to also dissolve in the aqueous phase. Examples of electrolytes include chlorides or sulfates of alkaline earth metals such as calcium or magnesium and chlorides or sulfates of alkali earth metals such as sodium. Such electrolyte can include a buffering agent for the control of pH during the polymerization, including such inorganic counterions as phosphate, borate, and carbonate, and mixtures thereof. Water soluble monomers may also be used in the aqueous phase, examples being acrylic acid and vinyl acetate.

Another component that may be present in the aqueous phase is a water-soluble free-radical initiator. The initiator can be present at up to about 20 mole percent based on the total moles of polymerizable monomers present in the oil phase. In certain embodiments, the initiator is present in an amount of from about 0.001 to about 10 mole percent based on the total moles of polymerizable monomers in the oil phase. Suitable initiators include ammonium persulfate, sodium persulfate, potassium persulfate, 2,2'-azobis(N,N'-dimethyleneisobutyramidine)dihydrochloride, and other suitable azo initiators. In certain embodiments, to reduce the potential for premature polymerization which may clog the emulsification system, addition of the initiator to the monomer phase may be just after or near the end of emulsification.

Photoinitiators present in the aqueous phase may be at least partially water soluble and can have between about 0.05% and about 10%, and in certain embodiments between about 0.2% and about 10% by weight of the aqueous phase. Lower amounts of photoinitiator allow light to better penetrate the HIPE foam, which can provide for polymerization deeper into the HIPE foam. However, if polymerization is done in an oxygen-containing environment, there should be enough photoinitiator to initiate the polymerization and overcome oxygen inhibition. Photoinitiators can respond rapidly and efficiently to a light source with the production of radicals, cations, and other species that are capable of initiating a polymerization reaction. The photoinitiators used in the present invention may absorb UV light at wavelengths of from about 200 nanometers (nm) to about 800 nm, in certain embodiments from about 200 nm to about 350 nm, and in certain embodiments from about 350 nm to about 450 nm. If the photoinitiator is in the aqueous phase, suitable types of water-soluble photoinitiators include benzophenones, benzils, and thioxanthones. Examples of photoinitiators include 2,2'-Azobis[2-(2-imidazolin-2-yl)propane]dihydrochloride; 2,2'-Azobis[2-(2-imidazolin-2-yl)propane]disulfate dehydrate; 2,2'-Azobis(1-imino-1-pyrrolidino-2-ethylpropane)dihydrochloride; 2,2'-Azobis[2-methyl-N-(2-hydroxyethyl)propionamide]; 2,2'-Azobis(2-methylpropionamidine)dihydrochloride; 2,2'-dicarboxymethoxydibenzalacetone, 4,4'-dicarboxymethoxydibenzalacetone, 4,4'-dicarboxymethoxydibenzalcyclohexanone,4-dimethylamino-4'-carboxymethoxydibenzalacetone; and 4,4'-disulphoxymethoxydibenzalacetone. Other suitable photoinitiators that can be used in the present invention are listed in U.S. Pat. No. 4,824,765 (Sperry et al.) issued Apr. 25, 1989.

In addition to the previously described components other components may be included in either the aqueous or oil phase of a HIPE. Examples include antioxidants, for example hindered phenolics, hindered amine light stabilizers; plasticizers, for example dioctyl phthalate, dinonyl sebacate; flame retardants, for example halogenated hydrocarbons, phosphates, borates, inorganic salts such as antimony trioxide or ammonium phosphate or magnesium hydroxide; dyes and pigments; fluorescers; filler pieces, for example starch, titanium dioxide, carbon black, or calcium carbonate; fibers; chain transfer agents; odor absorbers, for example activated carbon particulates; dissolved polymers; dissolved oligomers; and the like.

The heterogeneous mass comprises enrobeable elements and discrete pieces of foam. The enrobeable elements may be a web such as, for example, nonwoven, a fibrous structure, an air-laid web, a wet laid web, a high loft nonwoven, a needlepunched web, a hydroentangled web, a fiber tow, a woven web, a knitted web, a flocked web, a spunbond web, a layered spunbond/ melt blown web, a carded fiber web, a coform web of cellulose fiber and melt blown fibers, a coform web of staple fibers and melt blown fibers, and layered webs that are layered combinations thereof.

The enrobeable elements may be, for example, conventional absorbent materials such as creped cellulose wadding, fluffed cellulose fibers, wood pulp fibers also known as airfelt, and textile fibers. The enrobeable elements may also be fibers such as, for example, synthetic fibers, thermoplastic particulates or fibers, tricomponent fibers, and bicomponent fibers such as, for example, sheath/core fibers having the following polymer combinations: polyethylene/polypropylene, polyethylvinyl acetate/polypropylene, polyethylene/polyester, polypropylene/polyester, copolyester/polyester, and the like. The enrobeable elements may be any combination of the materials listed above and/or a plurality of the materials listed above, alone or in combination.

The enrobeable elements may be hydrophobic or hydrophilic. In an embodiment, the enrobeable elements may be treated to be made hydrophobic. In an embodiment, the enrobeable elements may be treated to become hydrophilic.

The constituent fibers of the heterogeneous mass can be comprised of polymers such as polyethylene, polypropylene, polyester, and blends thereof. The fibers can be spunbound fibers. The fibers can be meltblown fibers. The fibers can comprise cellulose, rayon, cotton, or other natural materials or blends of polymer and natural materials. The fibers can also comprise a super absorbent material such as polyacrylate or any combination of suitable materials. The fibers can be monocomponent, bicomponent, and/or biconstituent, non-round (e.g., capillary channel fibers), and can have major cross-sectional dimensions (e.g., diameter for round fibers) ranging from 0.1-500 microns. The constituent fibers of the nonwoven precursor web may also be a mixture of different fiber types, differing in such features as chemistry (e.g. polyethylene and polypropylene), components (mono- and bi-), denier (micro denier and >20 denier), shape (i.e. capillary and round) and the like. The constituent fibers can range from about 0.1 denier to about 100 denier.

In one aspect, known absorbent web materials in an as-made can be considered as being homogeneous throughout. Being homogeneous, the fluid handling properties of the absorbent web material are not location dependent, but are substantially uniform at any area of the web. Homogeneity can be characterized by density, basis weight, for example, such that the density or basis weight of any particular part of the web is substantially the same as an average density or basis weight for the web. By the apparatus and method of the present invention, homogeneous fibrous absorbent web materials are modified such that they are no longer homogeneous, but are heterogeneous, such that the fluid handling properties of the web material are location dependent. Therefore, for the heterogeneous absorbent materials of the present invention, at discrete locations the density or basis weight of the web may be substantially different than the average density or basis weight for the web. The heterogeneous nature of the absorbent web of the present invention permits the negative aspects of either of permeability or capillarity to be minimized by rendering discrete portions highly permeable and other discrete portions to have high capillarity. Likewise, the tradeoff between permeability and capillarity is managed such that delivering relatively higher permeability can be accomplished without a decrease in capillarity.

In an embodiment, the heterogeneous mass may also include superabsorbent material that imbibe fluids and form hydrogels. These materials are typically capable of absorbing large quantities of body fluids and retaining them under moderate pressures. The heterogeneous mass can include such materials dispersed in a suitable carrier such as cellulose fibers in the form of fluff or stiffened fibers.

In an embodiment, the heterogeneous mass may include thermoplastic particulates or fibers. The materials, and in particular thermoplastic fibers, can be made from a variety of thermoplastic polymers including polyolefins such as polyethylene (e.g., PULPEX.RTM.) and polypropylene, polyesters, copolyesters, and copolymers of any of the foregoing.

Depending upon the desired characteristics, suitable thermoplastic materials include hydrophobic fibers that have been made hydrophilic, such as surfactant-treated or silica-treated thermoplastic fibers derived from, for example, polyolefins such as polyethylene or polypropylene, polyacrylics, polyamides, polystyrenes, and the like. The surface of the hydrophobic thermoplastic fiber can be rendered hydrophilic by treatment with a surfactant, such as a nonionic or anionic surfactant, e.g., by spraying the fiber with a surfactant, by dipping the fiber into a surfactant or by including the surfactant as part of the polymer melt in producing the thermoplastic fiber. Upon melting and resolidification, the surfactant will tend to remain at the surfaces of the thermoplastic fiber. Suitable surfactants include nonionic surfactants such as Brij 76 manufactured by ICI Americas, Inc. of Wilmington, Del., and various surfactants sold under the Pegosperse.RTM. trademark by Glyco Chemical, Inc. of Greenwich, Conn. Besides nonionic surfactants, anionic surfactants can also be used. These surfactants can be applied to the thermoplastic fibers at levels of, for example, from about 0.2 to about 1 g. per sq. of centimeter of thermoplastic fiber.

Suitable thermoplastic fibers can be made from a single polymer (monocomponent fibers), or can be made from more than one polymer (e.g., bicomponent fibers). The polymer comprising the sheath often melts at a different, typically lower, temperature than the polymer comprising the core. As a result, these bicomponent fibers provide thermal bonding due to melting of the sheath polymer, while retaining the desirable strength characteristics of the core polymer.

Suitable bicomponent fibers for use in the present invention can include sheath/core fibers having the following polymer combinations: polyethylene/polypropylene, polyethylvinyl acetate/polypropylene, polyethylene/polyester, polypropylene/polyester, copolyester/polyester, and the like. Particularly suitable bicomponent thermoplastic fibers for use herein are those having a polypropylene or polyester core, and a lower melting copolyester, polyethylvinyl acetate or polyethylene sheath (e.g., DANAKLON.RTM., CELBOND.RTM. or CHISSO.RTM. bicomponent fibers). These bicomponent fibers can be concentric or eccentric. As used herein, the terms "concentric" and "eccentric" refer to whether the sheath has a thickness that is even, or uneven, through the cross-sectional area of the bicomponent fiber. Eccentric bicomponent fibers can be desirable in providing more compressive strength at lower fiber thicknesses. Suitable bicomponent fibers for use herein can be either uncrimped (i.e. unbent) or crimped (i.e. bent). Bicomponent fibers can be crimped by typical textile means such as, for example, a stuffer box method or the gear crimp method to achieve a predominantly two-dimensional or "flat" crimp.

The length of bicomponent fibers can vary depending upon the particular properties desired for the fibers and the web formation process. Typically, in an airlaid web, these thermoplastic fibers have a length from about 2mm to about 12mm long, or from about 2.5mm to about 7.5mm long, or from about 3.0mm to about 6.0mm long. The properties-of these thermoplastic fibers can also be adjusted by varying the diameter (caliper) of the fibers. The diameter of these thermoplastic fibers is typically defined in terms of either denier (grams per 9000 meters) or decitex (grams per 10,000 meters). Suitable bicomponent thermoplastic fibers as used in an airlaid making machine can have a decitex in the range from about 1.0 to about 20, or from about 1.4 to about 10, or from about 1.7 to about 7 decitex.

The compressive modulus of these thermoplastic materials, and especially that of the thermoplastic fibers, can also be important. The compressive modulus of thermoplastic fibers is affected not only by their length and diameter, but also by the composition and properties of the polymer or polymers from which they are made, the shape and configuration of the fibers (e.g., concentric or eccentric, crimped or uncrimped), and like factors. Differences in the compressive modulus of these thermoplastic fibers can be used to alter the properties, and especially the density characteristics, of the respective thermally bonded fibrous matrix.

The heterogeneous mass can also include synthetic fibers that typically do not function as binder fibers but alter the mechanical properties of the fibrous webs. Synthetic fibers include cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. These might include, for example, polyester fibers such as polyethylene terephthalate (e.g., DACRON.RTM. and KODEL.RTM.), high melting crimped polyester fibers (e.g., KODEL.RTM. 431 made by Eastman Chemical Co.) hydrophilic nylon (HYDROFIL.RTM.), and the like. Suitable fibers can also hydrophilized hydrophobic fibers, such as surfactant-treated or silica-treated thermoplastic fibers derived from, for example, polyolefins such as polyethylene or polypropylene, polyacrylics, polyamides, polystyrenes, polyurethanes and the like. In the case of nonbonding thermoplastic fibers, their length can vary depending upon the particular properties desired for these fibers. Typically they have a length from about 0.3 to 7.5 cm, or from about 0.9 to about 1.5 cm. Suitable nonbonding thermoplastic fibers can have a decitex in the range of about 1.5 to about 35 decitex, or from about 14 to about 20 decitex.

However structured, the total absorbent capacity of the heterogeneous mass containing foam pieces should be compatible with the design loading and the intended use of the mass. For example, when used in an absorbent article, the size and absorbent capacity of the heterogeneous mass may be varied to accommodate different uses such as incontinence pads, pantiliners, regular sanitary napkins, or overnight sanitary napkins. The heterogeneous mass can also include other optional components sometimes used in absorbent webs. For example, a reinforcing scrim can be positioned within the respective layers, or between the respective layers, of the heterogeneous mass.

The heterogeneous mass comprising open-cell foam pieces produced from the present invention may be used as an absorbent core or a portion of an absorbent core in absorbent articles, such as feminine hygiene articles, for example pads, pantiliners, and tampons; disposable diapers; incontinence articles, for example pads, adult diapers; homecare articles, for example wipes, pads, towels; and beauty care articles, for example pads, wipes, and skin care articles, such as used for pore cleaning.

In one embodiment the heterogeneous mass may be used as an absorbent core for an absorbent article. In such an embodiment, the absorbent core can be relatively thin, less than about 5 mm in thickness, or less than about 3 mm, or less than about 1 mm in thickness. Cores having a thickness of greater than 5 mm are also contemplated herein. Thickness can be determined by measuring the thickness at the midpoint along the longitudinal centerline of the pad by any means known in the art for doing while under a uniform pressure of 0.25 psi (172368 Pa). The absorbent core can comprise absorbent gelling materials (AGM), including AGM fibers, as is known in the art.

The heterogeneous mass may be formed or cut to a shape, the outer edges of which define a periphery. Additionally, the heterogeneous mass may be continuous such that it may be rolled or wound upon itself, with or without the inclusion of preformed cut lines demarcating the heterogeneous mass into preformed sections.

When used as an absorbent core, the shape of the heterogeneous mass can be generally rectangular, circular, oval, elliptical, or the like. Absorbent core can be generally centered with respect to the longitudinal centerline and transverse centerline of an absorbent article. The profile of absorbent core can be such that more absorbent is disposed near the center of the absorbent article. For example, the absorbent core can be thicker in the middle, and tapered at the edges in a variety of ways known in the art.

Components of the disposable absorbent article (i.e., diaper, disposable pant, adult incontinence article, sanitary napkin, pantiliner, etc.) described in this specification can at least partially be comprised of bio-sourced content as described in US 2007/0219521A1 Hird et al published on September 20, 2007, US 2011/0139658A1 Hird et al published on June 16, 2011, US 2011/0139657A1 Hird et al published on June 16, 2011, US 2011/0152812A1 Hird et al published on June 23, 2011, US 2011/0139662A1 Hird et al published on June 16, 2011, and US 2011/0139659A1 Hird et al published on June 16, 2011. These components include, but are not limited to, topsheet nonwovens, backsheet films, backsheet nonwovens, side panel nonwovens, barrier leg cuff nonwovens, super absorbent, nonwoven acquisition layers, core wrap nonwovens, adhesives, fastener hooks, and fastener landing zone nonwovens and film bases.

In at least one embodiment, a disposable absorbent article component comprises a bio-based content value from about 10% to about 100% using ASTM D6866-10, method B, in another embodiment, from about 25% to about 75%, and in yet another embodiment, from about 50% to about 60% using ASTM D6866-10, method B.

In order to apply the methodology of ASTM D6866-10 to determine the bio-based content of any disposable absorbent article component, a representative sample of the disposable absorbent article component must be obtained for testing. In at least one embodiment, the disposable absorbent article component can be ground into particulates less than about 20 mesh using known grinding methods (e.g., Wiley® mill), and a representative sample of suitable mass taken from the randomly mixed particles.

In at least one embodiment, a foam piece or an enrobeable element comprises a bio-based content value from about 10% to about 100% using ASTM D6866-10, method B, in another embodiment, from about 25% to about 75%, and in yet another embodiment, from about 50% to about 60%. Foam pieces may be made from bio-based content such as monomers as described in US2012/0108692 A1 Dyer published May 3, 2012.

In order to apply the methodology of ASTM D6866-10 to determine the bio-based content of any foam piece or enrobeable element, a representative sample of the foam piece or the enrobeable element must be obtained for testing. In at least one embodiment, the foam piece or the enrobeable element can be ground into particulates less than about 20 mesh using known grinding methods (e.g., Wiley® mill), and a representative sample of suitable mass taken from the randomly mixed particles.

### Validation of Polymers Derived from Renewable Resources

A suitable validation technique is through ¹⁴C analysis. A small amount of the carbon dioxide in the atmosphere is radioactive. This ¹⁴C carbon dioxide is created when nitrogen is struck by an ultra-violet light produced neutron, causing the nitrogen to lose a proton and form carbon of molecular weight 14 which is immediately oxidized to carbon dioxide. This radioactive isotope represents a small but measurable fraction of atmospheric carbon. Atmospheric carbon dioxide is cycled by green plants to make organic molecules during photosynthesis. The cycle is completed when the green plants or other forms of life metabolize the organic molecules, thereby producing carbon dioxide which is released back to the atmosphere. Virtually all forms of life on Earth depend on this green plant production of organic molecules to grow and reproduce. Therefore, the ¹⁴C that exists in the atmosphere becomes part of all life forms, and their biological products. In contrast, fossil fuel based carbon does not have the signature radiocarbon ratio of atmospheric carbon dioxide.

Assessment of the renewably based carbon in a material can be performed through standard test methods. Using radiocarbon and isotope ratio mass spectrometry analysis, the bio-based content of materials can be determined. ASTM International, formally known as the American Society for Testing and Materials, has established a standard method for assessing the bio-based content of materials. The ASTM method is designated ASTM D6866-10.

The application of ASTM D6866-10 to derive a "bio-based content" is built on the same concepts as radiocarbon dating, but without use of the age equations. The analysis is performed by deriving a ratio of the amount of organic radiocarbon (¹⁴C) in an unknown sample to that of a modern reference standard. The ratio is reported as a percentage with the units "pMC" (percent modern carbon).

The modern reference standard used in radiocarbon dating is a NIST (National Institute of Standards and Technology) standard with a known radiocarbon content equivalent approximately to the year AD 1950. AD 1950 was chosen since it represented a time prior to thermo-nuclear weapons testing which introduced large amounts of excess radiocarbon into the atmosphere with each explosion (termed "bomb carbon"). The AD 1950 reference represents 100 pMC.

"Bomb carbon" in the atmosphere reached almost twice normal levels in 1963 at the peak of testing and prior to the treaty halting the testing. Its distribution within the atmosphere has been approximated since its appearance, showing values that are greater than 100 pMC for plants and animals living since AD 1950. It's gradually decreased over time with today's value being near 107.5 pMC. This means that a fresh biomass material such as corn could give a radiocarbon signature near 107.5 pMC.

Combining fossil carbon with present day carbon into a material will result in a dilution of the present day pMC content. By presuming 107.5 pMC represents present day biomass materials and 0 pMC represents petroleum derivatives, the measured pMC value for that material will reflect the proportions of the two component types. A material derived 100% from present day soybeans would give a radiocarbon signature near 107.5 pMC. If that material was diluted with 50% petroleum derivatives, for example, it would give a radiocarbon signature near 54 pMC (assuming the petroleum derivatives have the same percentage of carbon as the soybeans).

A biomass content result is derived by assigning 100% equal to 107.5 pMC and 0% equal to 0 pMC. In this regard, a sample measuring 99 pMC will give an equivalent bio-based content value of 92%.

Assessment of the materials described herein can be done in accordance with ASTM D6866. The mean values quoted in this report encompasses an absolute range of 6% (plus and minus 3% on either side of the bio-based content value) to account for variations in end-component radiocarbon signatures. It is presumed that all materials are present day or fossil in origin and that the desired result is the amount of biobased component "present" in the material, not the amount of biobased material "used" in the manufacturing process.

The heterogeneous mass may serve as any portion of an absorbent article. In an embodiment, the heterogeneous mass may serve as the absorbent core of an absorbent article. In an embodiment, the heterogeneous mass may serve as a portion of the absorbent core of an absorbent article. In an embodiment, more than one heterogeneous mass may be combined wherein each heterogeneous mass differs from at least one other heterogeneous mass in either the choice of enrobeable elements or by a characteristic of its open-cell foam pieces. The different two or more heterogeneous masses may be combined to form an absorbent core. The absorbent article may further comprise a topsheet and a backsheet.

In an embodiment, the heterogeneous mass may be used as a topsheet for an absorbent article. The heterogeneous mass may be combined with an absorbent core or may only be combined with a backsheet.

In an embodiment, the heterogeneous mass may be combined with any other type of absorbent layer such as, for example, a layer of cellulose, a layer comprising superabsorbent gelling materials, a layer of absorbent airlaid fibers, or a layer of absorbent foam. Other absorbent layers not listed are contemplated herein.

In an embodiment, the heterogeneous mass may be utilized by itself for the absorption of fluids without placing it into an absorbent article.

According to an embodiment, an absorbent article can comprise a liquid pervious topsheet. The topsheet suitable for use herein can comprise wovens, non-wovens, and/or three-dimensional webs of a liquid impermeable polymeric film comprising liquid permeable apertures. The topsheet for use herein can be a single layer or may have a multiplicity of layers. For example, the wearer-facing and contacting surface can be provided by a film material having apertures which are provided to facilitate liquid transport from the wearer facing surface towards the absorbent structure. Such liquid permeable, apertured films are well known in the art. They provide a resilient three-dimensional fibre-like structure. Such films have been disclosed in detail for example in US 3929135, US 4151240, US 4319868, US 4324426, US 4343314, US 4591523, US 4609518, US 4629643, US 4695422 or WO 96/00548.

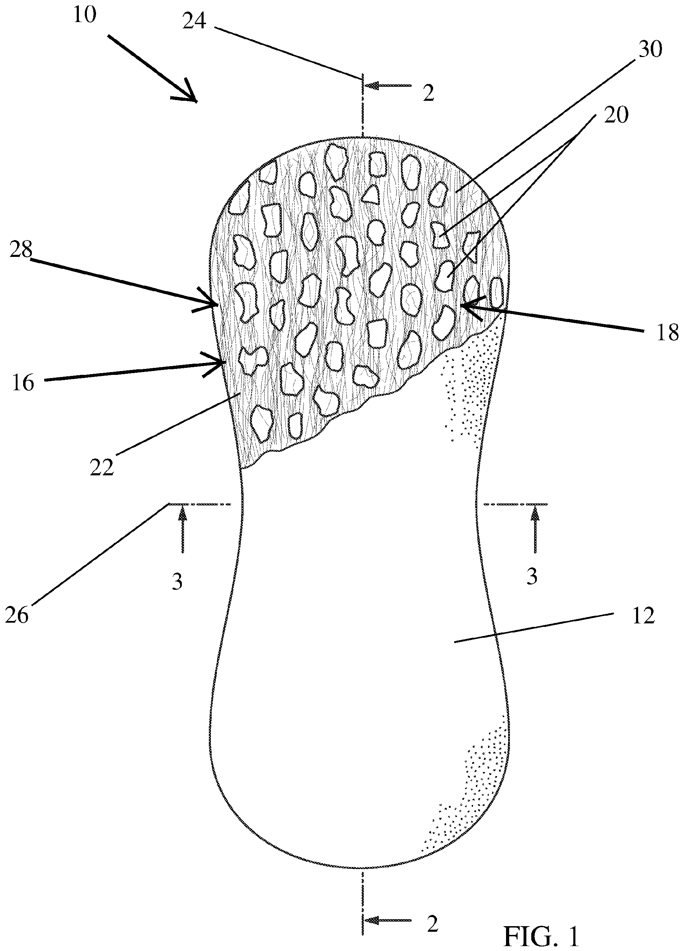

The absorbent articles of Figures 1 to 13 comprising embodiments of the heterogeneous mass can also comprise a backsheet and a topsheet. The backsheet may be used to prevent the fluids absorbed and contained in the absorbent structure from wetting materials that contact the absorbent article such as underpants, pants, pyjamas, undergarments, and shirts or jackets, thereby acting as a barrier to fluid transport. The backsheet according to an embodiment of the present invention can also allow the transfer of at least water vapour, or both water vapour and air through it.

Especially when the absorbent article finds utility as a sanitary napkin or panty liner, the absorbent article can be also provided with a panty fastening means, which provides means to attach the article to an undergarment, for example a panty fastening adhesive on the garment facing surface of the backsheet. Wings or side flaps meant to fold around the crotch edge of an undergarment can be also provided on the side edges of the napkin.

FIG. 1 is a plan view of a sanitary napkin 10 comprising a topsheet 12, a backsheet (not shown), an absorbent core 16 located between the topsheet 12 and the backsheet, a longitudinal axis 24, and a transverse axis 26. The absorbent core 16 comprises of a heterogeneous mass 18 comprising elements 30 and one or more discrete foam pieces 20 that enrobe the at least one element 30 of the heterogeneous mass 18. As shown in FIG.1 the elements 30 are fibers 22. A portion of the topsheet is cut out in order to show underlying portions.

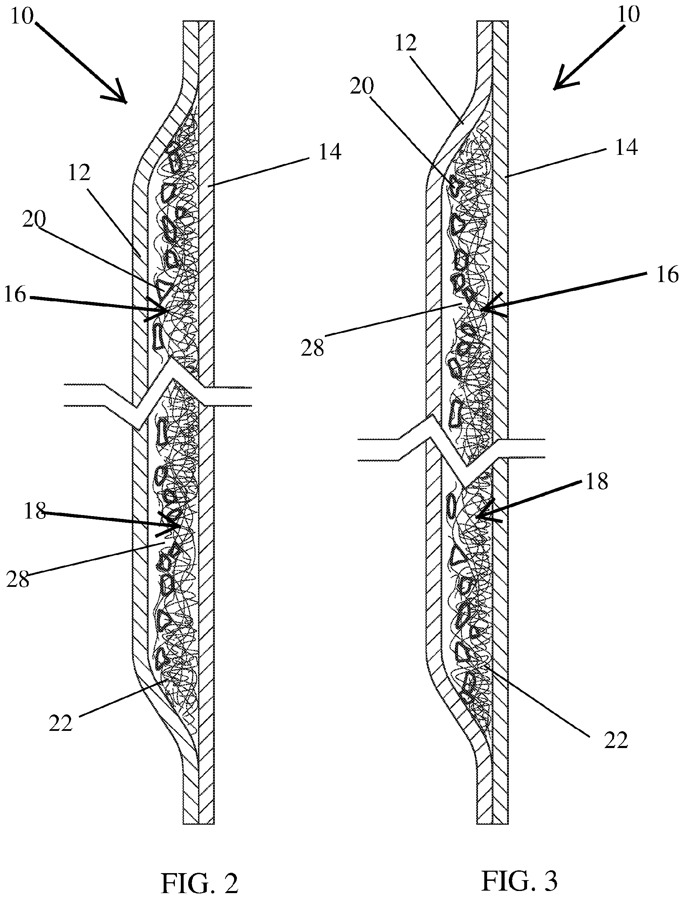

FIGS. 2 and 3 are cross sections of pad shown in FIG. 1, cut through the 2-2 vertical plane along the longitudinal axis 24 and cut through the 3-3 vertical plane along the transverse axis 26, respectively. As can be seen in FIGS. 2 and 3, the absorbent core 16 is between the topsheet 12 and the backsheet 14. As shown in the embodiment of FIGS. 2 and 3, the discrete foam pieces 20 are spread out throughout the absorbent core and enrobe the elements 30 of the heterogeneous mass 18. The discrete pieces 20 of foam may extend beyond the enrobeable elements to form part of the outer surface of the heterogeneous mass. Additionally, discrete pieces of foam may be fully intertwined within the heterogeneous mass of the absorbent core. Voids 28 containing gas are located between the fibers 22.

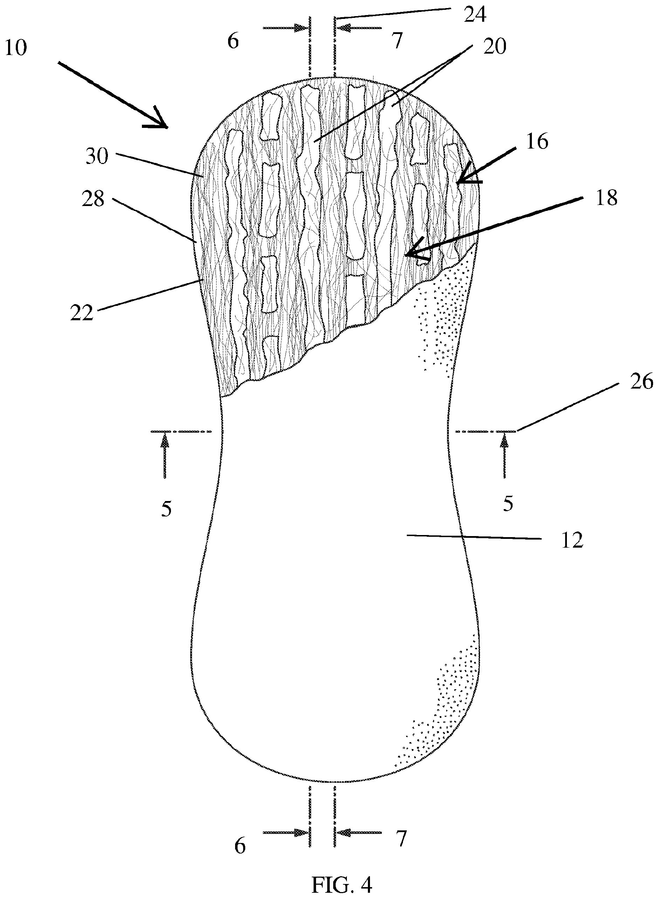

FIG. 4 is a plan view of a sanitary napkin 10 illustrating an embodiment of the invention. The sanitary napkin 10 comprises a topsheet 12, a backsheet (not shown), an absorbent core 16 located between the topsheet 12 and the backsheet, a longitudinal axis 24, and a transverse axis 26. The absorbent core 16 comprises of a heterogeneous mass 18 comprising elements 30 and one or more discrete foam pieces 20 that enrobe the at least one element 30 of the heterogeneous mass 18. As shown in FIG.4, the elements 30 are fibers 22. A portion of the topsheet is cut out in order to show underlying portions. As shown in FIG. 4 the discrete foam pieces 20 may be continuous along an axis of the heterogeneous mass, such as, for example, the longitudinal axis. Further, the discrete foam 20 may be arranged to form a line in the heterogeneous mass. The discrete foam pieces 20 are shown proximate to the top of the heterogeneous mass 18 but may also be located at any vertical height of the heterogeneous mass 18 such that enrobeable elements 30 may be located above and below the one or more of the discrete foam pieces 20.

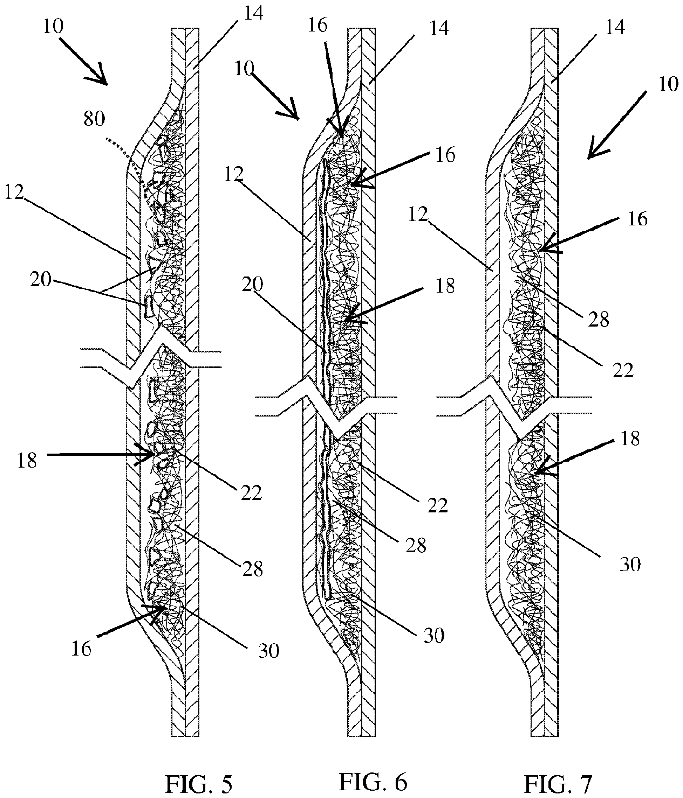

FIGS. 5, 6 and 7 are cross sections of the pad shown in FIG. 4, cut through the 5-5, the 6-6, and the 7-7 vertical planes, respectively. The 5-5 vertical plane is parallel to the transverse axis of the pad and the 6-6 and 7-7 vertical planes are parallel to the longitudinal axis. As can be seen in FIGS. 5 to 7, the absorbent core 16 is between the topsheet 12 and the backsheet 14. As shown in the embodiment of FIG. 5, the discrete foam pieces 20 are spread out throughout the absorbent core and enrobe the elements 30 of the heterogeneous mass 18. As shown in FIG. 6, a discrete foam piece 20 may be continuous and extend along the heterogeneous mass. As shown in FIG. 7, the heterogeneous mass may not have any discrete foam pieces along a line cross section of the absorbent core. Voids 28 containing gas are located between the fibers 22.

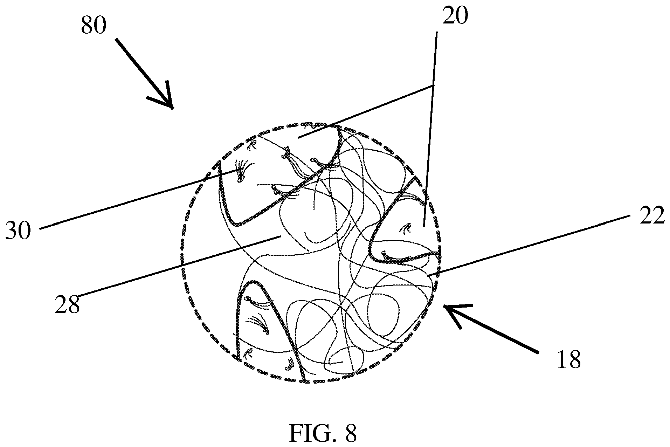

FIG. 8 is a zoomed in view of a portion of FIG. 5 indicated on FIG. 5 by a dotted line circle 80. As shown in FIG. 8, the heterogeneous mass 18 comprises discrete foam pieces 20 and enrobeable elements 30 in the form of fibers 22. Voids 28 containing gas are located between the fibers 22.

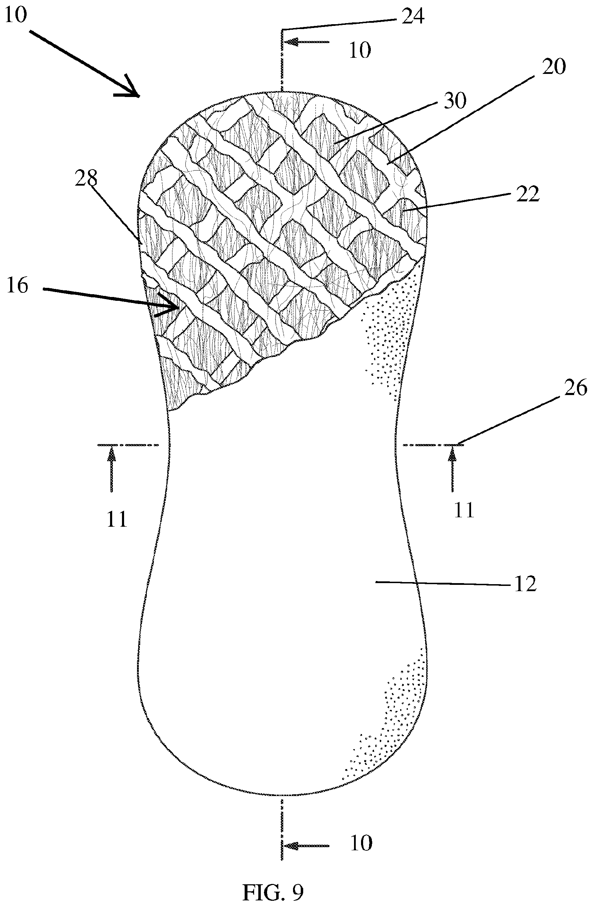

FIG. 9 is a plan view of a sanitary napkin 10 illustrating an embodiment of the invention. The sanitary napkin 10 comprises a topsheet 12, a backsheet (not shown), an absorbent core 16 located between the topsheet 12 and the backsheet, a longitudinal axis 24, and a transverse axis 26. The absorbent core 16 comprises of a heterogeneous mass 18 comprising elements 30 and one or more discrete foam pieces 20 that enrobe the at least one element 30 of the heterogeneous mass 18. As shown in FIG.9, the elements 30 are fibers 22. A portion of the topsheet is cut out in order to show underlying portions. As shown in FIG. 9, the discrete foam pieces 20 may form a pattern, such as, for example, a checkerboard grid.

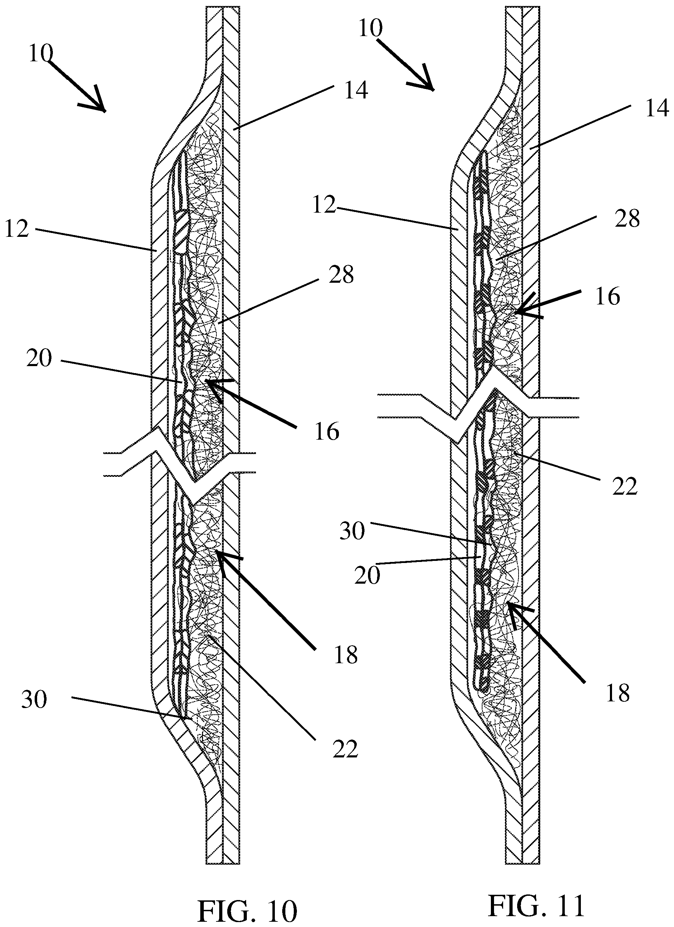

FIGS. 10 and 11 are cross sections of the pad shown in FIG. 9, cut through the 10-10 and 11-11 vertical planes, respectively. As can be seen in FIGS. 10 and 11, the absorbent core 16 is between the topsheet 12 and the backsheet 14. As shown in the embodiment of FIGS. 10 and 11, the discrete foam pieces 20 are spread out throughout the absorbent core and enrobe the elements 30 in the form of fibers 22 of the heterogeneous mass 18. Voids 28 containing gas are located between the fibers 22.

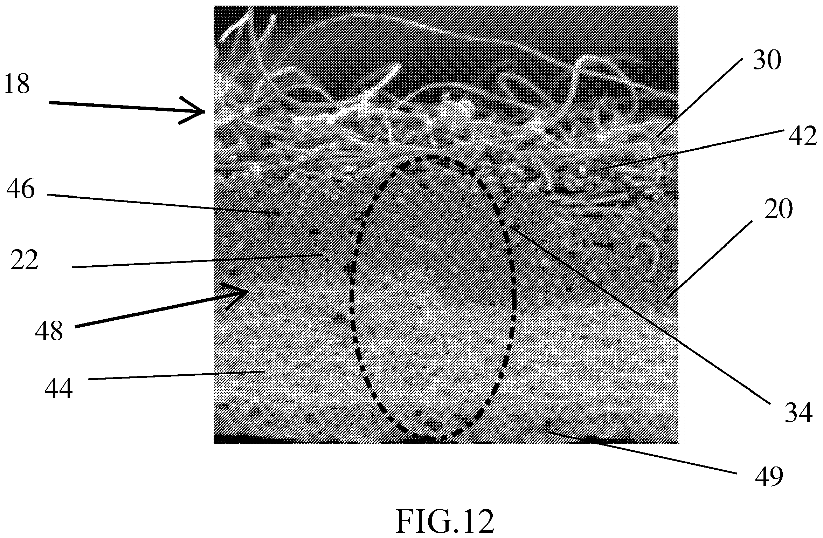

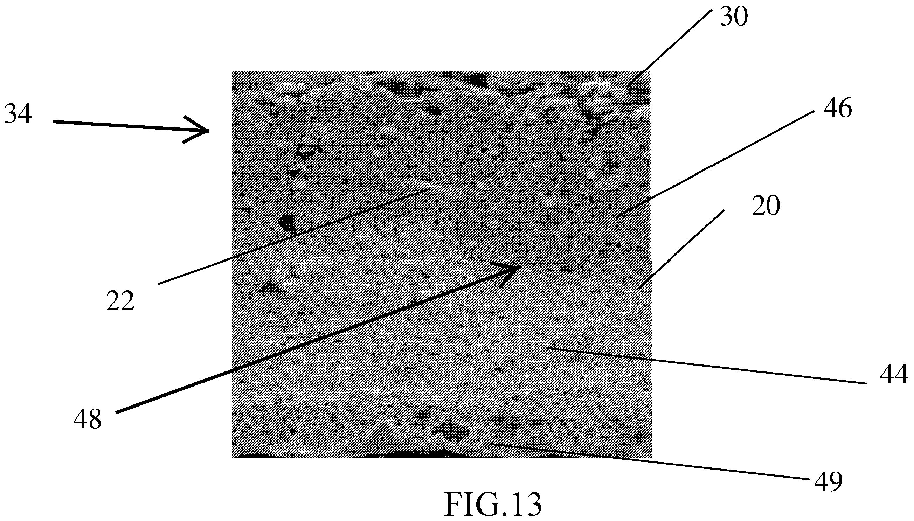

FIGS. 12 and 13 are cross-sectional SEM micrographs of a HIPE foam piece 20 intertwined within a heterogeneous mass 18 comprising nonwoven fibers 22.

FIG. 12 shows a SEM micrograph taken at 25x magnification. As shown in FIG. 12, there are three distinct pore-size ranges within three distinct zones of the heterogeneous mass. The enrobeable elements within the heterogeneous mass 18 have a first zone with a first distinct pore-size range 42. This zone is predominately enrobeable fibers. The HIPE foam piece of the heterogeneous mass has a second zone with a second distinct pore-size range 44. This second zone is predominately composed of HIPE foam. The HIPE foam piece of the heterogeneous mass has a third zone and a third pore-size range 46. This third zone is an intimate mixture of HIPE foam piece and enrobeable fibers. As shown in FIG. 12, a visually distinct demarcation 48 is formed within the single HIPE foam piece 20 between the second zone containing the second distinct pore-size range 44 and the third zone containing the third distinct pore-size range 46. Optionally, there can be a fourth zone within the heterogeneous mass with a fourth distinct pore-size range 49, shown at the bottom of Fig. 12. As shown in FIG. 12, a visually distinct demarcation 48 is formed within the single HIPE foam piece 20 between the second distinct pore size range 44 and the third distinct pore size range 46.

FIG. 13 shows a portion 34 of FIG. 12 taken at 100x magnification. As shown in FIG. 13, there are three distinct zones and three distinct pore-size ranges. The enrobeable elements within the heterogeneous mass 18 have a first zone with a first distinct pore-size range 42 at the top of the image. The HIPE foam piece of the heterogeneous mass has a second zone with a second distinct pore-size range 44 and a third zone and a third pore-size range 46. Optionally, there can be a fourth zone within the heterogeneous mass with a fourth distinct pore-size range 49, shown at the bottom of Fig. 13. As shown in FIG. 13, a visually distinct demarcation 48 is formed within the single HIPE foam piece 20 between the second distinct pore size range 44 and the third distinct pore size range 46.

As shown in FIGS. 12 and 13, when the zones are along a vertical axis, each zone has a relative height in relation to the overall heterogeneous mass height. The first zone may have a height that is between 1% and 50%, or from 2% to 40%, or from 3 to 25%, or from 5 to 15% of the overall heterogeneous mass height. Likewise, the second zone (HIPE only) may have a relative height to the overall heterogeneous mass height of between 5% to 75%, or from 10% to 60%, or from 15% to 50% or from 20 to 40% of the overall heterogeneous mass height. The third zone (mixture of HIPE foam piece and enrobeable fibers), may have a relative height to the overall heterogeneous mass height of between 10% and 100%, or between 15% and 75%, or between 20% and 50% of the overall heterogeneous mass height.

### Method for Assessing Areas for Pore Size Calculations Using SEM Imaging:

### Sample Preparation

The first step is to prepare the sample to be imaged using SEM: Section of the heterogeneous mass are cut into approximately 1.5 cm x 4 cm strips from the original samples. These strips are then cut the strips into sections. Each section should contain the entire composite. The strips should be cut using a razor blade, such as VWR Single Edge Industrial, 0.009" (ca. 0.23 mm) thick surgical carbon steel or equivalent, at room temperature (available from VWR Scientific, Radnor Pennsylvania, USA). Following the cutting of strips into sections, the sections are adhered to a mount using double-side Cu tape, with the-sectioned face up, and sputter Au coated.

### Analysis

Secondary Electron (SE) images are obtained using an SEM, such as a FEI Quanta 450 (available from FEI Company, Hillsboro, OR, USA), operated in high-vacuum mode using acceleration voltages between 3 and 5 kV and a working distance of approximately 12-18 mm. This methodology assumes the analyst is skilled in SEM operation so that images with sufficient contrast are obtained.

### Viewing the SEM Sample

Samples should be viewed at 25 or 50x magnification. The different pore-size ranges are distinguished by the different portions within the heterogeneous mass. Distinct portions exhibit different cell/pore sizes/open area/solid phase vs gas phase. The magnification for the portions is chosen to enable clear visualization of the portion and the ability to distinguish the solid phase from the gas phase.

Determination of portions having different pore-size ranges is done at a magnification of 25x. The heterogeneous mass SEM is divided into an upper portion and a lower portion at the point where the lowest fiber is located along the Z-direction. Each portion is then divided into three portions. This creates three portions with the first upper portion and the first lower portion sharing a boundary. The pore-size range of the upper second portion is compared to pore-size range of the lower second portion. The lower third region may be compared to the upper second region and the lower second region to determine if there is an additional pore-size range. The upper third region may be compared to the upper second region and the lower second region to determine if there is an additional pore-size range. Pore size ranges are determined using software that is capable of analyzing the SEM images.

## Claims

1. A heterogeneous mass (18) comprising a longitudinal axis (24), a lateral axis (26), a vertical axis, one or more enrobeable elements (30) and one or more discrete open-cell foam pieces (20) wherein the heterogeneous mass comprises of three or more pore-size ranges (42, 44, 46), wherein at least two of the pore-size ranges (42, 44) are in one or more of the open-cell foam pieces (20), **characterized in that** the open-cell foam is a thermoset polymeric foam made from polymerization of a High Internal Phase Emulsion (HIPE) formed by combining an aqueous phase and an oil phase in a ratio between about 8:1 and 140:1, and the one or more discrete open-cell foam pieces are polymerized around the enrobeable elements.

2. The heterogeneous mass of claim 1, wherein at least one discrete open-cell foam piece exhibits two distinct pore-size ranges, wherein a first pore-size range is between 100 nanometers and 100 microns and a second pore-size range is between 200 and 5000 microns.

3. The heterogeneous mass of claim 2, wherein the second pore-size average is at least three times the pore-size average of the first pore-size range.

4. The heterogeneous mass of any of claims 1 to 3, wherein the three or more pore-size ranges are found in three or more vertical distinct zones within the heterogeneous mass.

5. The heterogeneous mass of any of claims 1 to 4, wherein at least a first portion of an open-cell foam piece exhibits a first pore-size range average and a second portion of the open-cell foam piece exhibits a second pore-size range average, wherein the first pore-size range average does not overlap with the second pore-size range average.

6. A heterogeneous mass of any of claims 1 to 5, wherein the pore sizes change in size along one of the longitudinal axis, lateral axis, or vertical axis.

7. The heterogeneous mass of any of claims 1 to 6, wherein the open-cell foams change in size such that the pore-size average at a second end of an axis are a multiple of the pore-size average at a first end of an axis.

8. The heterogeneous mass of any of claims 1 to 7, wherein the heterogeneous mass comprises between about 5% and about 99% of discrete open-cell foam pieces for a fixed volume.

9. The heterogeneous mass of any of claims 1 to 8, wherein the first pore-size range is between 100 nanometers to 5,000 microns.

10. The heterogeneous mass of any of claims 1 to 9, wherein at least a pore of the open-cell foam contains a portion of an enrobeable element in the form of a fiber.

11. The heterogeneous mass of any of claims 1 to 10, wherein the heterogeneous mass comprises a plurality of discrete open-cell foam pieces and wherein the discrete open-cell foam pieces are profiled along an axis of the heterogeneous mass.

12. An absorbent article comprising an absorbent core comprising the heterogeneous mass of claim any of claims 1 to 11.

## Patentansprüche

1. Heterogene Masse (18), umfassend eine Längsachse (24), eine Querachse (26), eine vertikale Achse, ein oder mehrere umhüllbare Elemente (30) und ein oder mehrere diskrete offenzellige Schaumstoffstücke (20), wobei die heterogene Masse drei oder mehr Porengrößenbereiche (42, 44, 46) umfasst, wobei sich mindestens zwei der Porengrößenbereiche (42, 44) in einem oder mehreren offenzelligen Schaumstoffstücken (20) befinden, **dadurch gekennzeichnet, dass** der offenzellige Schaumstoff ein duroplastischer Polymerschaumstoff ist, der durch Polymerisation einer HIPE (High Internal Phase Emulsion) hergestellt wurde, die durch Kombinieren einer wässrigen Phase und einer Ölphase in einem Verhältnis zwischen etwa 8 : 1 und 140 : 1 geschäumt wurde, und dadurch, dass das eine oder die mehreren diskreten offenzelligen Schaumstoffstücke um die umhüllbaren Elemente herum polymerisiert sind.

2. Heterogene Masse nach Anspruch 1, wobei mindestens ein diskretes offenzelliges Schaumstoffstück zwei unterschiedliche Porengrößenbereiche aufweist, wobei ein erster Porengrößenbereich zwischen 100 Nanometern und 100 Mikrometern liegt und ein zweiter Porengrößenbereich zwischen 200 und 5000 Mikrometern liegt.

3. Heterogene Masse nach Anspruch 2, wobei der zweite Porengrößendurchschnitt mindestens das Dreifache des Porengrößendurchschnitts des ersten Porengrößenbereichs beträgt.

4. Heterogene Masse nach einem der Ansprüche 1 bis 3, wobei die drei oder mehr Porengrößenbereiche in drei oder mehr vertikalen, verschiedenen Zonen innerhalb der heterogenen Masse gefunden werden.

5. Heterogene Masse nach einem der Ansprüche 1 bis 4, wobei mindestens ein erster Abschnitt eines offenzelligen Schaumstoffstücks einen ersten Porengrößenbereichdurchschnitt aufweist und ein zweiter Abschnitt des offenzelligen Schaumstoffstücks einen zweiten Porengrößenbereichdurchschnitt aufweist, wobei der erste Porengrößenbereichdurchschnitt den zweiten Porengrößenbereichdurchschnitt nicht überschneidet.

6. Heterogene Masse nach einem der Ansprüche 1 bis 5, wobei sich die Porengrößen entlang einer von der Längsachse, Querachse oder vertikalen Achse in der Größe ändern.

7. Heterogene Masse nach einem der Ansprüche 1 bis 6, wobei sich die offenzelligen Schaumstoffe in der Größe so ändern, dass der Porengrößendurchschnitt an einem zweiten Ende einer Achse ein Vielfaches des Porengrößendurchschnitts an einem ersten Ende einer Achse betragen.

8. Heterogene Masse nach einem der Ansprüche 1 bis 7, wobei die heterogene Masse für ein festgelegtes Volumen zwischen etwa 5 % und etwa 99 % diskrete offenzellige Schaumstoffstücke umfasst.

9. Heterogene Masse nach einem der Ansprüche 1 bis 8, wobei der erste Porengrößenbereich zwischen 100 Nanometern und 5.000 Mikrometern liegt.

10. Heterogene Masse nach einem der Ansprüche 1 bis 9, wobei mindestens eine Pore des offenzelligen Schaumstoffs einen Abschnitt eines umhüllbaren Elements in Form einer Faser enthält.

11. Heterogene Masse nach einem der Ansprüche 1 bis 10, wobei die heterogene Masse eine Vielzahl von diskreten offenzelligen Schaumstoffstücken umfasst und wobei die diskreten offenzelligen Schaumstoffstücke entlang einer Achse der heterogenen Masse profiliert sind.

12. Absorptionsartikel, umfassend einen Absorptionskern, der die heterogene Masse nach einem der Ansprüche 1 bis 11 umfasst.

## Revendications

1. Masse hétérogène (18) comprenant un axe longitudinal (24), un axe latéral (26), un axe vertical, un ou plusieurs éléments enrobables (30) et une ou plusieurs pièces individuelles de mousse à alvéoles ouvertes (20) dans laquelle la masse hétérogène comprend trois plages de taille de pore ou plus (42, 44, 46), dans laquelle au moins deux des plages de taille de pore (42, 44) sont dans une ou plusieurs pièces de mousse à alvéoles ouvertes (20), **caractérisée en ce que** la mousse à alvéoles ouvertes est une mousse polymère thermodurcissable fabriquée par polymérisation d'une émulsion à phase interne élevée (HIPE) formée en combinant une phase aqueuse et une phase huileuse dans un rapport entre environ 8:1 et 140:1, et la ou les pièces individuelles de mousse à alvéoles ouvertes sont polymérisées autour des éléments enrobables.

2. Masse hétérogène selon la revendication 1, dans laquelle au moins une pièce individuelle de mousse à alvéoles ouvertes présente deux plages de taille de pore distinctes, dans laquelle une première plage de taille de pore est comprise entre 100 nanomètres et 100 micromètres et une deuxième plage de taille de pore est comprise entre 200 et 5000 micromètres.

3. Masse hétérogène selon la revendication 2, dans laquelle la moyenne de deuxième taille de pore vaut au moins trois fois la moyenne de taille de pore de la première plage de taille de pore.

4. Masse hétérogène selon l'une quelconque des revendications 1 à 3, dans laquelle les trois plages de taille de pore ou plus se trouvent dans trois zones distinctes verticales ou plus au sein de la masse hétérogène.

5. Masse hétérogène selon l'une quelconque des revendications 1 à 4, dans laquelle au moins une première partie d'une pièce de mousse à alvéoles ouvertes présente une première moyenne de plage de taille de pore et une deuxième partie de la pièce de mousse à alvéoles ouvertes présente une deuxième moyenne de plage de taille de pore, dans laquelle la première moyenne de plage de taille de pore ne chevauche pas la deuxième moyenne de plage de taille de pore.

6. Masse hétérogène selon l'une quelconque des revendications 1 à 5, dans laquelle les tailles de pore changent de taille le long d'un parmi l'axe longitudinal, l'axe latéral, ou l'axe vertical.

7. Masse hétérogène selon l'une quelconque des revendications 1 à 6, dans laquelle les mousses à alvéoles ouvertes changent de taille de telle sorte que la moyenne de taille de pore au niveau d'une deuxième extrémité d'un axe sont un multiple de la moyenne de taille de pore au niveau d'une première extrémité d'un axe.

8. Masse hétérogène selon l'une quelconque des revendications 1 à 7, dans laquelle la masse hétérogène comprend entre environ 5 % et environ 99 % de pièces individuelles de mousse à alvéoles ouvertes pour un volume fixe.

9. Masse hétérogène selon l'une quelconque des revendications 1 à 8, dans laquelle la première plage de taille de pore est comprise entre 100 nanomètres et 5000 micromètres.

10. Masse hétérogène selon l'une quelconque des revendications 1 à 9, dans laquelle au moins un pore de la mousse à alvéoles ouvertes contient une partie d'un élément enrobable sous la forme d'une fibre.

11. Masse hétérogène selon l'une quelconque des revendications 1 à 10, dans laquelle la masse hétérogène comprend une pluralité de pièces individuelles de mousse à alvéoles ouvertes et dans laquelle les pièces individuelles de mousse à alvéoles ouvertes sont profilées le long d'un axe de la masse hétérogène.

12. Article absorbant comprenant une âme absorbante comprenant la masse hétérogène selon l'une quelconque des revendications 1 à 11.
